(19) Europäisches Patentamt
European Patent Office
Office européen des brevets

(11) Veröffentlichungsnummer: **0 159 966**
**B1**

(12) # EUROPÄISCHE PATENTSCHRIFT

(45) Veröffentlichungstag der Patentschrift: **08.08.90**

(21) Anmeldenummer: **85810178.5**

(22) Anmeldetag: **22.04.85**

(51) Int. Cl.⁵: **C 07 D 405/12,** A 01 N 47/36 // C07D307/46, C07D307/54, C07D333/18

(54) **Sulfonylharnstoffe.**

(30) Priorität: **27.04.84 CH 2062/84**

(43) Veröffentlichungstag der Anmeldung: **30.10.85 Patentblatt 85/44**

(45) Bekanntmachung des Hinweises auf die Patenterteilung: **08.08.90 Patentblatt 90/32**

(84) Benannte Vertragsstaaten: **BE CH DE FR GB IT LI NL**

(56) Entgegenhaltungen:
**EP-A-0 085 476**
**EP-A-0 097 122**

(73) Patentinhaber: **CIBA-GEIGY AG**
**Klybeckstrasse 141**
**CH-4002 Basel (CH)**

(72) Erfinder: **Schurter, Rolf, Dr.**
**Holzmattstrasse 45**
**CH-4102 Binningen (CH)**
Erfinder: **Pissiotas, Georg, Dr.**
**Am Sonnenrain 71**
**D-7850 Lörrach (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue, herbizid wirksame und pflanzenwuchsregulierende N-(2-Heteropcyclyl-phenylsulfonyl)-N'-pyrimidinyl- und -triazinyl-harnstoffe, Verfahren zu ihrer Herstellung, die sie als Wirkstoffe enthaltenden Mittel sowie deren Verwendung zum Bekämpfen von Unkräutern, vor allem selektiv in Nutzpflanzenkulturen oder zum Regulieren und Hemmen des Pflanzenwachstums. Darüber hinaus betrifft die Erfindung auch als Zwischenprodukte hergestellte neue 2-Heterocyclylphenylsulfonylisocyanate, -isothiocyanate-, phenylsulfonylcarbamate sowie 2-Heterocyclylphenylsulfonamide.

Die erfindungsmegässen N-(2-Heterocyclyl-phenylsulfonyl)-N'-pyrimidinyl- und triazinyl-harnstoffe entsprechen der allgemeinen Formel I

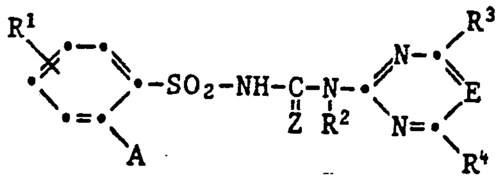

worin

A einen Rest

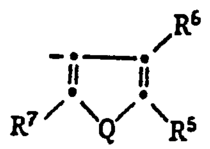

oder

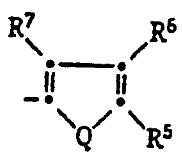

E Stickstoff oder die Methingruppe —CH=,

Q Sauerstoff, Schwefel oder die Iminogruppe, —NR$^8$=,

Z Sauerstoff oder Schwefel,

R$^1$ Wasserstoff, Halogen, Nitro, $C_1$—$C_4$-Alkyl, $C_1$—$C_4$-Halogenalkyl, $C_1$—$C_4$-Alkoxy, $C_1$—$C_4$-Halogenalkoxy, $C_1$—$C_4$-Alkoxycarbonyl, $C_1$—$C_4$-Alkylthio, $C_1$—$C_4$-Alkylsulfinyl, $C_1$—$C_4$-Alkylsulfonyl oder $C_2$—$C_5$-Alkoxyalkoxy,

R$^2$ Wasserstoff, $C_1$—$C_4$-Alkyl oder $C_1$—$C_3$-Alkoxy,

R$^3$ und R$^4$ unabhängig voneinander Wasserstoff, Halogen, $C_1$—$C_4$-Alkyl, $C_1$—$C_4$-Halogenalkyl, $C_3$—$C_5$-Cycloalkyl, $C_1$—$C_4$-Alkoxy, $C_1$—$C_4$-Halogenalkoxy, $C_1$—$C_4$-Alkylthio, $C_1$—$C_4$-Halogenalkylthio, $C_2$—$C_5$-Alkoxyalkyl, $C_3$—$C_6$-Dialkoxyalkyl oder einen Aminorest —NR$^{16}$R$^{17}$,

R$^5$ einen $C_1$—$C_6$-Alkylrest, der durch Hydroxy, $C_1$—$C_4$-Alkylthio, Cyan oder eine Gruppe —COOR$^{12}$ oder —NR$^{13}$R$^{14}$ substituiert ist, $C_3$—$C_6$-Alkoxyalkoxy oder $C_1$—$C_4$-Halogenalkoxy, $C_2$—$C_6$-Halogenalkenyl, einen Phenyl-, Benzyl-, Phenoxy- oder Phenylthiorest, welcher unsubstituiert oder durch $C_1$—$C_3$-Alkyl, $C_1$—$C_3$-Alkoxy oder Halogen substituiert ist, oder R$^5$ eine der Gruppierungen —COR$^{10}$, —C(R$^{10}$)=NOR$^{11}$, —CH=CH—COOR$^{12}$, —CH=C(COOR$^{12}$)$_2$, —CH=C(CN)—COOR$^{12}$, —CR$^{10}$(OR$^{14}$)$_2$,

$$-CH_2-C(R^{15})(NHCOR^{14})-COOR^{12},$$

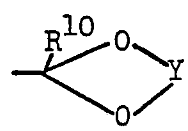

oder —NR$^{13}$—COR$^{14}$,

R$^6$ und R$^7$ unabhängig voneinander je Wasserstoff, Halogen $C_1$—$C_6$-Alkyl, $C_1$—$C_6$-Halogenalkyl, $C_2$—$C_6$-Alkoxyalkyl, $C_1$—$C_6$-Alkoxy, $C_1$—$C_4$-Alkylthio, Di($C_1$—$C_4$-alkyl)amino, Cyan, Nitro, Methoxycarbonyl oder dasselbe wie R$^5$ oder —COOR$^9$,

R$^8$ Wasserstoff, $C_1$—$C_6$-Alkyl, $C_3$—$C_6$-Cycloalkyl, Benzyl, Phenyl oder einen Rest —COOR$^{12}$,

R$^9$ einen $C_2$—$C_6$-Alkylrest, welcher unsubstituiert oder durch Halogen, $C_1$—$C_4$-Alkoxy oder Phenyl substituiert ist, $C_3$—$C_6$-Alkenyl oder $C_3$—$C_6$-Alkinyl,

R$^{10}$ Wasserstoff, $C_1$—$C_6$-Alkyl unsubstituiert oder substituiert durch Halogen, $C_1$—$C_4$-Alkoxy oder Phenoxy, welches seinerseits durch Halogen, $C_1$—$C_3$-Alkyl oder $C_1$—$C_3$-Alkoxy substituiert sein kann, oder $C_2$—$C_4$-Alkenyl,

R$^{11}$ Wasserstoff, $C_1$—$C_6$-Alkyl unsubstituiert oder substituiert durch Cyan oder —COOR$^{12}$,

R$^{12}$ Wasserstoff, Methyl, Halomethyl, Methoxymethyl, Benzyl oder dasselbe wie R$^9$,

R$^{13}$ und R$^{14}$ unabhängig voneinander je Wasserstoff, $C_1$—$C_6$-Alkyl oder $C_3$—$C_6$-Alkenyl,

R$^{15}$ Wasserstoff, $C_1$—$C_6$-Alkyl oder einen Rest —COOR$^{12}$,

R$^{16}$ und R$^{17}$ unabhängig voneinander je Wasserstoff oder $C_1$—$C_4$-Alkyl und

Y eine $C_2$—$C_8$-Alkylengruppe bedeuten,

sowie die Salze dieser Verbindungen mit organischen oder anorganischen Basen.

Harnstoffverbindungen, Triazinverbindungen und Pyrimidinverbindungen mit herbizider Wirkung sind allgemein bekannt. Kürzlich wurden N-(Heterocyclyl-aminocarbonyl)-arylsulfonamidverbindungen mit herbizider und pflanzenwuchsregulierender Wirkung, beispielsweise im US-Patent Nr. 4 127 405 und in den europäischen Patentanmeldungen 44807, 44808, 51465 und 85476 beschrieben.

In den Definitionen ist unter Alkyl geradkettiges oder verzweigtes Alkyl zu verstehen; z.B.: Methyl, Aethyl, n-Propyl, i-Propyl oder die vier isomeren Butyl. Cycloalkylreste sind z.B. Cyclopropyl, Cyclobutyl und Cyclopentyl.

Unter Alkoxy ist zu verstehen: Methoxy, Aethoxy, n-Propyloxy, i-Propyloxy, die vier isomeren Butyloxyreste, n-Amyloxy, i-Amyloxy, 2-Amyloxy oder 3-Amyloxy, insbesondere aber Methoxy, Aethoxy oder i-Propoxy.

Beispiele für Alkylthio sind Methylthio, Aethylthio, n-Propylthio, i-Propylthio, n-Butylthio oder n-Pentylthio, insbesondere aber Methylthio und Aethylthio.

Unter Halogen in den Definitionen sowie als Teil in Halogenalkoxy sind Fluor, Chlor, Brom und Jod, vorzugsweise jedoch Fluor und Chlor zu verstehen.

Die Wirkstoffe umfassen ebenfalls die Salze, die die Verbindungen der Formel I mit Aminen, Alkali- und Erdalkalimetalbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre alphatische und aromatische Amine wie Methylamin, Aethylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Die durch das Symbol A gekennzeichneten Reste sind über die 2- oder 3-Stellung gebundene Furyl-, Thienyl- und Pyrrolderivate die noch durch Reste $R^5$, $R^6$, $R^7$ oder im Fall von Pyrrol auch $R^8$ substituiert sind.

Unter den Sulfonylharnstoffen der Formel I wirken diejenigen besonders gut, welche der Formel Ia entsprechen,

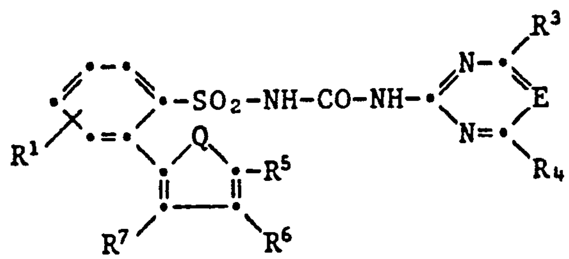

(Ia)

worin E, Q, $R^1$, $R^3$, $R^4$, $R^5$, $R^6$, $R^7$ die unter Formel I gegebene Bedeutung haben, vor allem diejenigen, in denen

Q Sauerstoff oder Schwefel,

$R^1$ Wasserstoff oder Halogen,

$R^3$ Methyl, Methoxy, Aethoxy, Chlor, Methoxymethyl, Dimethoxymethyl, $C_1$—$C_2$-Halogenalkoxy oder Dimethylamino

$R^4$ Methyl, Methoxy, Aethoxy, Cyclopropyl oder $OCHF_2$

während E und $R^5$, $R^6$ und $R^7$ die unter Formel I angegebenen Bedeutungen haben.

Besonders aufgefallen sind Verbindungen der Formel Ib

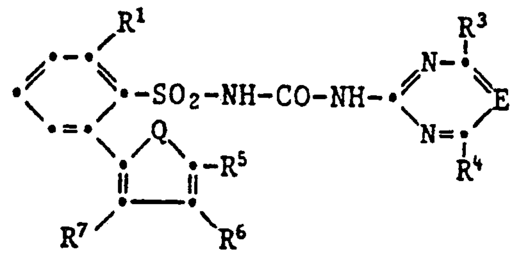

(Ib)

worin

Q Sauerstoff oder Schwefel

$R^1$ Wasserstoff oder Chlor

$R^3$ und $R^4$ unabhängig voneinander je Methyl oder Methoxy bedeuten während E, $R^5$, $R^6$ und $R^7$ die unter Formel I gegebene Bedeutung haben, speziell

N-[2-(5-Acetyl-furan-2-yl)-phenylsulfonyl]-N'-(4,6-dimethoxypyrimidin-2-yl)-harnstoff und

N-[2-(5-Acetyl-furan-2-yl)-phenylsulfonyl-N'-(4-methoxy-6-methylpyrimidin-2-yl)-harnstoff.

Weiterhin aufgefallen sind Sulfonylharnstoffe der Formel Ic

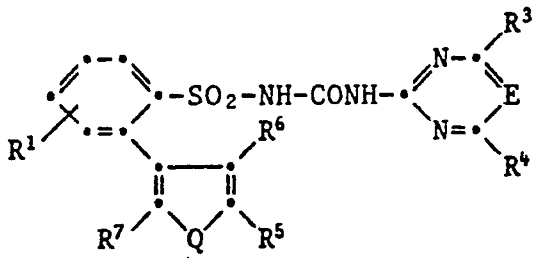

(Ic)

worin E, Q, $R^1$, $R^3$, $R^4$, $R^5$, $R^6$ und $R^7$ die unter Formel I gegebene Bedeutung haben, vor allem diejenigen, in denen

Q Sauerstoff oder Schwefel,

$R^1$ Wasserstoff oder Halogen,

$R^3$ Methyl, Methoxy, Aethoxy, Chlor, Methoxymethyl, Dimethoxymethyl, $C_1$—$C_2$-Halogenalkoxy oder Dimethylamino

$R^4$ Methyl, Methoxy, Aethoxy, Cyclopropyl oder $OCF_2$ während E, $R^5$, $R^6$ und $R^7$ die unter der Formel I gegebenen Bedeutungen haben.

Ebenfalls gute Wirkung zeigen die Verbindungen der Formel Id

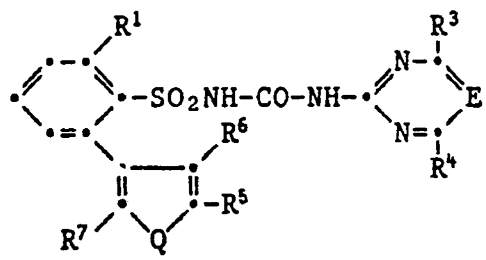

(Id)

worin

Q Sauerstoff oder Schwefel

$R^1$ Wasserstoff oder Chlor

$R^3$ und $R^4$ unabhängig voneinander je Methyl oder Methoxy bedeuten, während E, $R^5$, $R^6$ und $R^7$ die unter Formel I gegebenen Bedeutung haben, speziell

N-[2-(5-Acetylfuran-2-methyl-3-yl)-phenylsulfonyl]-N'-(4,6-dimethoxypyrimidin-2-yl)-harnstoff und

N-[2-(5-Acetylfuran-2-methyl-3-yl)-phenylsulfonyl]-N'-(4-methoxy-6-methylpyrimidin-2-yl)-harnstoff.

Die Herstellung der Verbindung der Formel I erfolgt in einem inerten, organischen Lösungsmittel.

Nach einem ersten Verfahren werden die Verbindungen der Formel I erhalten, indem man ein 2-Heterocyclyl-phenylsulfonamid der Formel II

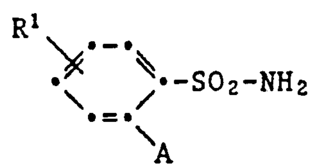

(II),

worin $R^1$ und A die unter Formel I gegebene Bedeutung haben, gegebenenfalls in Gegenwart einer Base mit einem N-Pyrimidinyl- oder -triazinylcarbamat der Formel III

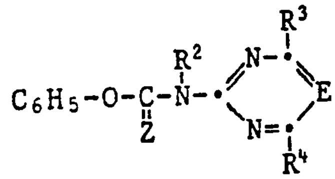

(III),

worin

E, $R^2$, $R^3$, $R^4$ und Z die unter Formel I gegebene Bedeutung haben, umsetzt.

Nach einem zweiten Verfahren gelangt man zu Verbindungen der Formel I, indem man ein 2-Heterocyclyl-phenylsulfonylisocyanat oder -isothiocyanat der Formel IV

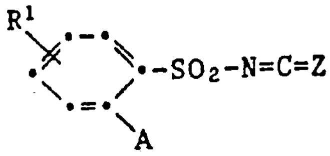

(IV),

worin A, $R^1$ und Z die unter Formel I gegebene Bedeutung haben, gegebenenfalls in Gegenwart einer Base mit einem Aminopyrimidin oder -triazin der Formel V

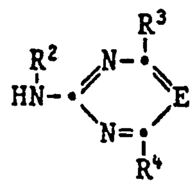

(V)

worin

E, $R^2$, $R^3$ und $R^4$ die unter Formel I gegebenen Bedeutung haben, umsetzt.

Schliesslich kann man die Verbindungen der Formel I auch erhalten, indem man ein 2-Heterocyclyl-phenylsulfonylcarbamat der Formel VI

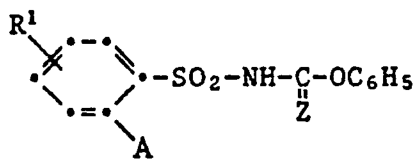

(VI)

worin A, $R^1$ und Z die unter Formel I gegebene Bedeutung haben, mit einem Aminopyrimidin oder -triazin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze überführt werden. Dieses geschieht beispielsweise durch Umsetzen mit der äquimolaren Menge Base und Verdampfen des Lösungsmittels.

Die Ausgangsstoffe der Formeln II, IV und VI sind neu und können nach den folgenden Methoden hergestellt werden:

Die neuen und als Zwischenprodukte verwendeten substituierten Phenylsulfonamide der Formel II werden z.B. aus den entsprechenden Anilin-Derivaten durch Diazotierung und Austausch der Diazogruppe mit Schwefeldioxid in Gegenwart eines Katalysators wie Kupfer-I-chlorid in Salzsäure oder Essigsäure und Umsetzen des entstandenen substituierten Phenylsulfonylchlorids mit Ammoniak erhalten.

Entsprechende Anilin-Derivate sind bekannt oder nach bekannten Methoden herstellbar.

Die Zwischenprodukte der Formeln II, IV, VI sind neu und speziell für die Synthese von Verbindungen oder Formel I entwickelt worden. Sie bilden deshalb weitere Gegenstände der vorliegenden Erfindung.

Die substituierten Phenylsulfonylisocyanate der Formel IV können durch Umsetzung der Sulfonamide der Formel II mit Phosgen in Anwesenheit von Butylisocyanat in einem inerten, organischen Lösungsmittel bei Rückflusstemperatur erhalten werden, Aehnliche Herstellungsmethoden sind in "Neuere Methoden der präparativen organischen Chemie", Band VI, 211—229, Verlag Chemie, Weinheim, 1970, beschrieben.

Die substituierten Isothiocyanate der Formel IV werden durch Behandlung der Sulfonamide der Formel II mit Schwefelkohlenstoff und Kaliumhydroxid und anschliessender Umsetzung des Dikaliumsalzes mit Phosgen erhalten. Solche Verfahren sind in Arch. Pharm. 299, 174 (1966) beschrieben.

Die substituierten Phenylsulfonylcarbamate der Formel VI werden durch Umsetzung der Sulfonamide der Formel II mit Diphenylcarbonat oder Chlorameisensäurephenylester oder deren Schwefelanalogen in Gegenwart einer Base hergestellt. Aehnliche Verfahren sind in der japanischen Patentschrifft 61169 erwähnt.

Die als Ausgangsmaterialien verwendeten Aminopyrimidine und -triazine der Formel V sowie entsprechende Phenylcarbamate der Formel III sind entweder seit langem bekannt oder in der schweizerischen Patentanmeldung Nr. 3527/82—8 beschrieben oder sie lassen sich nach bekannten Methoden aus dort beschriebenen Verbindungen erhalten.

Die Umsetzungen zu Verbindungen der Formel I werden vorteilhafterweise in aprotischen, inerten, organischen Lösungsmitteln oder Lösungsmittelgemischen vorgenommen. Geeignete Lösungsmittel sind Aether wie Dioxan, Diäthyläther, Tetrahydrofuran, Aethylenglykoldimethyläther, Diäthylenglykoldimethyläther; Kohlenwasserstoffe wie Benzol, Toluol, Xylol, Cyclohexan; Nitrile wie Acetonitril, Propionitril; chlorierte Kohlenwasserstoffe wie Methylenchlorid, Chloroform, Tetrachlorkohlenstoff, Chlorbenzol oder Dimethylsulfoxid. Die Reaktionstemperaturen liegen vorzugsweise zwischen −20°C und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit das Reaktionsgemisch aufgewärmt. Die Reaktionszeiten können ebenfalls durch Zugabe von Base als Reaktionskatalysator verkürzt werden.

Als Basen können sowohl organische Basen wie Trimethylamin, Triäthylamin, Chinuclidin, Pyridin, 1,4-Diazabicyclo[2.2.2]octan, 1,8-Diazabicyclo[5.4.0]undec-7-en etc. als auch anorganische Basen wie Hydride wie Natrium- oder Calciumhybrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte können durch Einengen des Lösungsmittels isoliert und durch Umkristallisieren oder Zerreiben des festen Rückstandes in Lösungsmitteln, in denen sie sich nicht gut lösen, wie Aether oder aromatischen Kohlenwasserstoffen gereinigt werden.

Die Wirkstoffe der Formel I sind stabile Verbindungen. Ihre Handhabung bedarf keiner vorsorglichen Massnahmen.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei — im Vergleich zu anderen Herbiziden und Wuchsregulatoren — sehr geringen Aufwandmengen.

Die Verbindungen der Formel I haben ausserdem starke pflanzenwuchsregulierende, insbesondere pflanzenwuchshemmende Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute können, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwuchsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B.

mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzgut die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyl-äther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden.

Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensid- gemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$—$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazol-derivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierbar gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäueester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulerungstechnik gebrächlichen Tenside sind u.a. in folgenden Publikationen beschrieben:

"Mc Cutcheon's Detergents and Emulsifiers Annual", MC Publishing Corp., Ridgewood, New Jersey, 1979.

M. and J. Ash, "Encyclopedia of Surfactants", Vol. I—III, Chemical Publishing Co., New York, 1980—1981.

H. Stache, "Tensid-Taschenbuch", 2. Aufl., C. Hanser Verlag, München, Wien, 1981;

Diese Zubereitungen enthalten in der Regel 0,1 bis 95%, insbesondere 0,1 bis 80% Wirkstoff der

Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent).

| | |
|---|---|
| *Emulgierbare Konzentrate:* | |
| Aktiver Wirkstoff: | 1 bis 20%, bevorzugt 5 bis 10% |
| oberflächenaktive Mittel: | 5 bis 30%, vorzugsweise 10 bis 20% |
| flüssiges Trägermittel: | 50 bis 94%, vorzugsweise 70 bis 85% |
| *Stäbe:* | |
| Aktiver Wirkstoff: | 0,1 bis 10%, vorzugsweise 0,1 bis 1% |
| festes Trägermittel: | 99,9 bis 90%, vorzugsweise 99,9 bis 99% |
| *Suspensions-Konzentrate:* | |
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30% |
| *Benetzbares Pulver:* | |
| Aktiver Wirkstoff: | 0,5 bis 90%, vorzugsweise 1 bis 80% |
| oberflächenaktives Mittel: | 0,5 bis 20%, vorzugsweise 1 bis 15% |
| festes Trägermittel: | 5 bis 95% vorzugsweise 15 bis 90% |
| *Granulate:* | |
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hibaub zu 0,001% an Wirkstoff verdünnt werden.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derart formulierte Mittel sind Bestandteile dieser Erfindung.

Das nachfolgende Beispiel dient zur näheren Erläuterung der Erfindung.

Beispiel 1

Herstellung von N-[2-(5-Acetyl-furan-2-yl)-phenylsulfonyl]-N'-(4,6-dimethoxy-pyrimidin-2-yl)-harnstoff

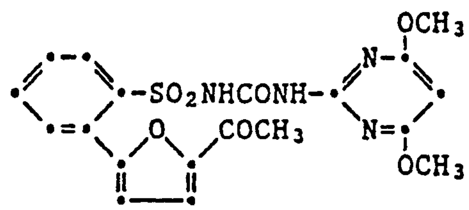

Man suspendiert 2,15 g (0.008 mol) 2-(5-Acetyl-furanyl-2-yl)-phenylsulfonamid und 2,20 g 4,6-Dimethoxy-2-phenoxycarbonylamino-pyrimidin in 40 ml Acetonitril. Dann lässt man 1,25 ml 1,8-Diazabicyclo[5.4.0]undec-7-en in 5 ml Acetonitril langsam zutropfen, wobei allmählich eine Lösung entsteht. Man rührt 3 Studen bei Raumtemperatur. Das Reaktionsgemisch wird auf Eis/Wasser gegossen und mit 0,5 ml Methansulfonsäure versetzt. Das Gemisch wird 30 Minuten gerührt, der entstandene Niederschlag wird abfiltriert, mit Wasser und Diäthyläther gewaschen und getrocknet. Man erhält 3,5 g (98% der Theorie) des obigen Harnstoffes. Schmelzpunkt 199—200°C.

In analoger Weise zu diesem Beispiel werden folgende Harnstoffe hergestellt:

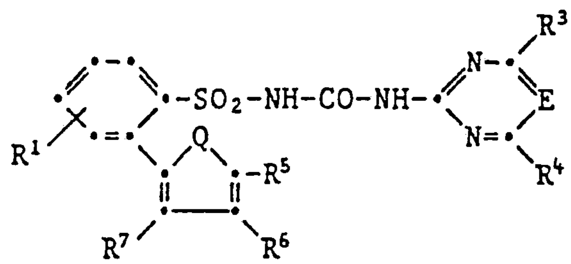

TABELLE 1

| Nr. | Q | $R^1$ | $R^5$ | $R^6$ | $R^7$ | E | $R^3$ | $R^4$ | Smp. [°C] |
|---|---|---|---|---|---|---|---|---|---|
| 1.01 | O | H | $COCH_3$ | H | H | CH | $OCH_3$ | $OCH_3$ | 199—200 Z |
| 1.02 | O | H | $COCH_3$ | H | H | CH | $OCH_3$ | $CH_3$ | 213—215 Z |
| 1.03 | O | H | $COCH_3$ | H | H | N | $OCH_3$ | $OCH_3$ | |
| 1.04 | O | H | $COCH_3$ | H | H | N | $OCH_3$ | $CH_3$ | |
| 1.05 | S | H | $COCH_3$ | H | H | CH | $OCH_3$ | $OCH_3$ | |
| 1.06 | S | H | $COCH_3$ | H | H | N | $OCH_3$ | $OCH_3$ | |
| 1.07 | O | 6-Cl | $COCH_3$ | H | H | N | $OCH_3$ | $OCH_3$ | |
| 1.08 | O | H | $CH_2SCH_3$ | H | H | CH | $OCH_3$ | $OCH_3$ | |
| 1.09 | O | H | $CH_2SCH_3$ | H | H | N | $OCH_3$ | $OCH_3$ | |
| 1.10 | O | H | $-C(CH_3)=NOCH_3$ | H | H | CH | $OCH_3$ | $OCH_3$ | |
| 1.11 | O | H | $-C(CH_3)=NOCH_3$ | H | H | N | $OCH_3$ | $OCH_3$ | |
| 1.12 | O | H | $-C(CH_3)=NOCH_2CN$ | H | H | CH | $OCH_3$ | $OCH_3$ | |
| 1.13 | O | H | $-CH=CH-COOCH_3$ | H | H | CH | $OCH_3$ | $OCH_3$ | |
| 1.14 | S | H | $-CH=CH-COOCH_3$ | H | H | CH | $OCH_3$ | $OCH_3$ | |
| 1.15 | O | H | $-CH_2CN$ | H | H | N | $OCH_3$ | $OCH_3$ | |
| 1.16 | O | H | $-CH_2COOC_2H_5$ | H | H | CH | $OCH_3$ | $OCH_3$ | |
| 1.17 | S | H | $-CH_2COOCH_3$ | H | H | CH | $OCH_3$ | $OCH_3$ | |
| 1.18 | O | H | $-CH_2N(CH_3)_2$ | H | H | CH | $OCH_3$ | $OCH_3$ | |
| 1.19 | O | H | 2-Methyl-1-3-dioxolan-2-yl | H | H | CH | $OCH_3$ | $OCH_3$ | |
| 1.20 | O | H | 2-Methyl-1,3-dioxolan-2-yl | H | H | N | $OCH_3$ | $OCH_3$ | |
| 1.21 | O | H | 1,3-Dioxolan-2-yl | H | H | CH | $OCH_3$ | $OCH_3$ | |
| 1.22 | O | H | CHO | H | H | CH | $OCH_3$ | $OCH_3$ | 179 Z |
| 1.23 | O | H | CHO | H | H | CH | $CH_3$ | $OCH_3$ | 171 Z |
| 1.24 | O | H | CHO | H | H | N | $CH_3$ | $OCH_3$ | 175 Z |
| 1.25 | O | H | $-CH_2CN$ | H | H | CH | $OCH_3$ | $OCH_3$ | 190—193 |

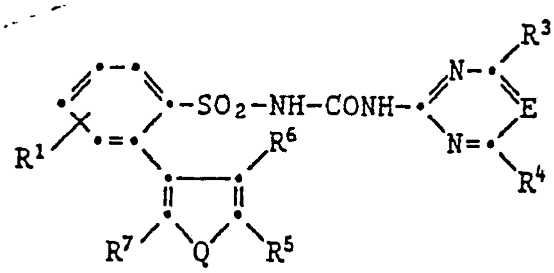

TABELLE 2

| Nr. | Q | $R^1$ | $R^5$ | $R^6$ | $R^7$ | E | $R^3$ | $R^4$ | Smp. [°C] |
|---|---|---|---|---|---|---|---|---|---|
| 2.01 | S | H | $COCH_3$ | H | $CH_3$ | CH | $OCH_3$ | $CH_3$ | |
| 2.02 | S | H | $COCH_3$ | H | $CH_3$ | CH | $OCH_3$ | $OCH_3$ | |
| 2.03 | S | H | $COCH_3$ | H | $CH_3$ | N | $OCH_3$ | $OCH_3$ | |
| 2.04 | O | H | $COCH_3$ | H | $CH_3$ | CH | $OCH_3$ | $OCH_3$ | |
| 2.05 | O | H | $COCH_3$ | H | $CH_3$ | N | $OCH_3$ | $OCH_3$ | |
| 2.06 | O | 6-Cl | $COCH_3$ | H | $CH_3$ | CH | $OCH_3$ | $OCH_3$ | |
| 2.07 | S | 6-Cl | $COCH_3$ | H | $CH_3$ | N | $OCH_3$ | $OCH_3$ | |
| 2.08 | O | H | $COCH_3$ | $CH_3$ | $CH_3$ | CH | $OCH_3$ | $OCH_3$ | |
| 2.09 | O | H | $COCH_3$ | $CH_3$ | $CH_3$ | N | $OCH_3$ | $OCH_3$ | |

Zwischenprodukte:

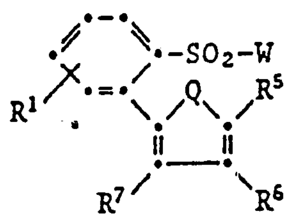

TABELLE 3

| Nr. | Q | $R^1$ | $R^5$ | $R^6$ | $R^7$ | W | Phys. Daten |
|---|---|---|---|---|---|---|---|
| 3.01 | O | H | $-COCH_3$ | H | H | $-NH_2$ | Smp. 183—186°C |
| 3.02 | S | H | $-COCH_3$ | H | H | $-NH_2$ | |
| 3.03 | O | 6-Cl | $-COCH_3$ | H | H | $-NH_2$ | |
| 3.04 | S | 6-Cl | $-COCH_3$ | H | H | $-NH_2$ | |
| 3.05 | O | H | $-CH_2SCH_3$ | H | H | $-NH_2$ | |
| 3.06 | O | 6-Cl | $-CH_2SCH_3$ | H | H | $-NH_2$ | |
| 3.07 | S | H | $-CH_2SCH_3$ | H | H | $-NH_2$ | |
| 3.08 | O | H | $-C(CH_3)=NOCH_3$ | H | H | $-NH_2$ | |
| 3.09 | O | H | $-C(CH_3)=NOCH_2CN$ | H | H | $-NH_2$ | |
| 3.10 | O | H | $-CH=CH-COOCH_3$ | H | H | $-NH_2$ | |
| 3.11 | S | H | $-CH=CH-COOCH_3$ | H | H | $-NH_2$ | |
| 3.12 | O | H | $-CH_2-CN$ | H | H | $-NH_2$ | Smp. 155°C |
| 3.13 | O | H | $-CH_2COOC_2H_5$ | H | H | $-NH_2$ | |
| 3.14 | S | H | $-CH_2COOCH_3$ | H | H | $-NH_2$ | |
| 3.15 | O | H | $-CH_2-N(CH_3)_2$ | H | H | $NH_2$ | |
| 3.16 | O | H | $-CH=C(COOCH_3)_2$ | H | H | $NH_2$ | |
| 3.17 | O | H | $-N(CH_3)COCH_3$ | H | H | $NH_2$ | |
| 3.18 | O | H | $-CH=C(CN)COOCH_3$ | H | H | $NH_2$ | |
| 3.19 | NH | H | $CH_2COOC_2H_5$ | H | H | $NH_2$ | |
| 3.20 | O | H | $CH_2COOCH_3$ | H | H | Cl | |
| 3.21 | S | H | $CH_2COOCH_3$ | H | H | Cl | |
| 3.28 | O | H | CHO | H | H | $NH_2$ | Smp. 110—115°C |
| 3.29 | O | H | 2-Methyl-1,3-dioxolan-2-yl | H | H | $NH_2$ | |

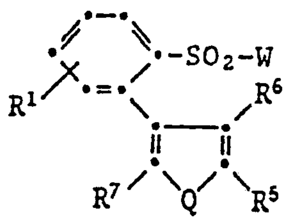

TABELLE 4

| Nr. | Q | $R^1$ | $R^5$ | $R^6$ | $R^4$ | W | phys. Daten |
|---|---|---|---|---|---|---|---|
| 4.01 | S | H | $-COCH_3$ | H | $CH_3$ | $NH_2$ | |
| 4.02 | O | H | $-COCH_3$ | H | $CH_3$ | $NH_2$ | |
| 4.03 | S | 6-Cl | $-COCH_3$ | H | $CH_3$ | $NH_2$ | |
| 4.04 | O | H | $-COCH_3$ | $CH_3$ | $CH_3$ | $NH_2$ | |

Formulierungsbeispiele

Beispiel 3

Formulierungsbeispiele für flüssige Wirkstoffe der Formel I (% = Gewichtsprozent)

| a) Emulsionskonzentrate | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | — | — |
| Tributylphenoyl-polyäthylenglykoläther (30 Mol AeO) | — | 12% | 4,2% |
| Cyclohexanon | — | 15% | 20% |
| Xylolgemisch | 70% | 25% | 20% |

Aus solchen Konzentraten können durch Verdünnen mit Wasser Emulsionen jeder gewünschten Konzentration hergestellt werden.

| b) Lösungen | a) | b) | c) | d) |
|---|---|---|---|---|
| Wirkstoff | 80% | 10% | 5% | 95% |
| Aethylenglykol-monomethyl-äther | 20% | — | — | — |
| Polyäthylenglykol MG 400 | — | 70% | — | — |
| N-Methyl-2-pyrrolidon | — | 20% | — | — |
| Epoxidiertes Kobosnussöl | — | — | 1% | 5% |
| Benzin (Siedegrenzen 160—190°C) | — | — | 94% | — |

Die Lösungen sind zur Anwendung in Form kleinster Tropfen geeignet.

c) Granulate

| | a) | b) |
|---|---|---|
| Wirkstoff | 5% | 10% |
| Kaolin | 94% | — |
| Hochdisperse Kieselsäure | 1% | — |
| Attapulgit | — | 90% |

Der Wirkstoff wird in Methylenchlorid gelöst, auf den Träger aufgesprüht und das Lösungsmittel anschliessend im Vakuum abgedampft.

d) Stäubemittel

| | a) | b) |
|---|---|---|
| Wirkstoff | 2% | 5% |
| Hochdisperse Kieselsäure | 1% | 5% |
| Talkum | 97% | — |
| Kaolin | — | 90% |

Durch inniges Vermischen der Trägerstoffe mit dem Wirkstoff erhält man gebrauchsfertige Stäubemittel.

Beispiel 4
Formulierungsbeispiele für feste Wirkstoffe der Formel I (% = Gewichtsprozent)

a) Spritzpulver

| | a) | b) |
|---|---|---|
| Wirkstoff | 20% | 60% |
| Na-Ligninsulfonat | 5% | 5% |
| Na-Laurylsulfat | 3% | — |
| Na-Diisobutylnaphthalinsulfonat | — | 6% |
| Octylphenolpolyäthylenglykoläther (7—8 Mol AeO) | — | 2% |
| Hochdisperse Kieselsäure | 5% | 27% |
| Kaolin | 67% | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | |
|---|---|
| Wirkstoff | 10% |
| Octylphenolpolyäthylenglykoläther (4—5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 5% | 8% |
| Talkum | 95% | — |
| Kaolin | — | 92% |

Man erhält anwendungsfertige Stäubemittel, indem der Wirkstoff mit dem Träger vermischt und auf einer geeigneten Mühle vermahlen wird.

| d) Extruder-Granulat | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | |
|---|---|
| Wirkstoff | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel 5
Herbizidwirkung vor dem Auflaufen der Pflanzen

Kunststofftöpfe werden mit expandiertem Vermiculit ((Dichte: 0,135 g/cm$^3$, Wasserabsorptionsvermögen: 0,565 1/1) gefüllt, Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sangguinalis. Die Versuchsgefässe werden anschliessend in einer Klimakammer bei 20°C, einer Beleuchtung von ca. 20 kLux und einer relativen Luftfeuchtigkeit von 70% ghealten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5% eines handelsüblichen Flüssigdüngers zugesetzt. 12 Tage nach der Aussaat wird der Versuch ausgewertet und die Wirkung auf die Versuchspflanzen nach dem folgenden Massatab bewertet:

1 Pflanze abgestorben oder hat nicht gekeimt
2,3 sehr schwere Schäden
4,5 schwere Schäden, Pflanze verkümmert
6 Schäden, die die Pflanze nach Abklingen der phytotosischen Wirkung regenerieren kann,
7,8 leichte Schäden
9 Pflanze gedeiht wie unbehandelte Kontrollpflanzen.

Die Resultate sind in der Tabelle 5 zusammengefasst.

TABELLE 5

| Wirkstoff | 1.01 | 1.02 | 1.27 | 1.28 | 1.29 | 1.30 |
|---|---|---|---|---|---|---|
| Pflanze: | | | | | | |
| Nasturtium | 2 | 2 | 1 | 1 | 1 | 1 |
| Agrostis | 2 | 2 | 1 | 1 | 1 | 1 |
| Stellaria media | 2 | 2 | 1 | 1 | 1 | 1 |
| Digitaria sanguinalis | 2 | 2 | 1 | 1 | 1 | 1 |

Beispiel 6
Wuchshemmung bei Getreide

In Kunststofftöpfen mit sterilisierter Ende werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwaches (60—90% der Kontrolle), sowie teilweise die Zunahme der Stengeldurchmesser auf.

Beispiel 7
Wuchshemmung bei Gräsern

In Kunststoffschalen mit Erde-Torf-Sand-Gemisch (6:3:1) werden die Gräser Lolium perenne, Poa pratensis, Festucca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Application wird das Wachstum der Gräser beurteilt.

Die Verbindungen der Formel I bewirken die Reduzierung des Neuzuwachses von 10—30% im Vergleich zur unbehandelten Kontrolle.

**Patentansprüche**

1. N-(2-Heterocyclyl-phenylsulfonyl)-N'-pyrimidinyl- und triazinyl-harnstoffe der Formel I

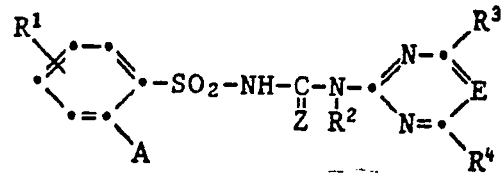

worin

A einen Rest

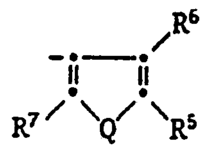 oder 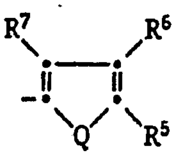

E Stickstoff oder die Methingruppe —CH=,
Q Sauerstoff, Schwefel oder die Iminogruppe $-NR^8$,
Z. Sauerstoff oder Schwefel,
$R^1$ Wasserstoff, Halogen, Nitro, $C_1-C_4$-Alkyl, $C_1-C_4$-Halogenalkyl, $C_1-C_4$-Alkoxy, $C_1-C_4$-Halogenalkoxy, $C_1-C_4$-Alkoxycarbonyl, $C_1-C_4$-Alkylthio, $C_1-C_4$-Alkylsulfinyl, $C_1-C_4$-Alkylsulfonyl oder $C_2-C_5$-Alkoxyalkoxy,
$R^2$ Wasserstoff, $C_1-C_4$-Alkyl oder $C_1-C_3$-Alkoxy,
$R^3$ und $R^4$ unabhängig voneinander Wasserstoff, Halogen, $C_1-C_4$-Alkyl, $C_1-C_4$-Halogenalkyl, $C_3-C_5$-Cycloalkyl, $C_1-C_4$-Alkoxy, $C_1-C_4$-Halogenalkoxy, $C_1-C_4$-Alkylthio, $C_1-C_4$-Halogenalkylthio, $C_2-C_5$-Alkoxyalkyl, $C_3-C_6$-Dialkoxyalkyl oder einen Aminorest $-NR^{16}R^{17}$,
$R^5$ einen $C_1-C_6$-Alkylrest, der durch Hydroxy, $C_1-C_4$-Alkylthio, Cyan oder eine Gruppe $-COOR^{12}$ oder $-NR^{13}R^{14}$ substituiert ist, $C_2-C_6$-Alkoxyalkoxy oder $C_1-C_4$-Halogenalkoxy, $C_2-C_6$-Halogenalkenyl, einen Phenyl-, Benzyl, Phenoxy- oder Phenylthiorest, welcher unsubstituiert oder durch $C_1-C_3$-Alkyl, $C_1-C_3$-Alkoxy oder Halogen substituiert ist, oder $R^5$ eine der Gruppierungen $-COR^{10}$, $-C(R^{10})=NOR^{11}$, $-CH=CH-COOR^{12}$, $-CH=C(COOR^{12})_2$, $-CH=C(CN)-COOR^{12}$, $-CR^{10}(OR^{14})_2$,

$-CH_2-C(R^{15})-COOR^{12}$,
NHCOR[14]

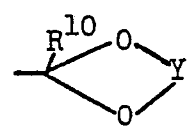

oder $-NR^{14}-COR^{14}$,
$R^6$ und $R^7$ unabhängig voneinander je Wasserstoff, Halogen, $C_1-C_6$-Alkyl, $C_1-C_6$-Halogenalkyl, $C_2-C_6$-Alkoxyalkyl, $C_1-C_6$-Alkoxy, $C_1-C_4$-Alkylthio, Di($C_1-C_4$-alkyl)amino, Cyan, Nitro, Methoxycarbonyl oder dasselbe wie $R^5$ oder $-COOR^9$,
$R^8$ Wasserstoff, $C_1-C_6$-Alkyl, $C_3-C_6$-Cycloalkyl, Benzyl, Phenyl oder einen Rest $-COOR^{12}$,
$R^9$ einen $C_2-C_6$-Alkylrest, welcher unsubstituiert oder durch Halogen, $C_1-C_4$-Alkoxy oder Phenyl substituiert ist, $C_3-C_6$-Alkenyl oder $C_3-C_6$-Alkinyl,
$R^{10}$ Wasserstoff, $C_1-C_6$-Alkyl unsubstituiert oder substituiert durch Halogen, $C_1-C_4$-Alkoxy oder Phenoxy, welches seinerseits durch Halogen, $C_1-C_3$-Alkyl oder $C_1-C_3$-Alkoxy substituiert sein kann, oder $C_2-C_4$-Alkenyl,
$R^{11}$ Wasserstoff, $C_1-C_6$-Alkyl unsubstituiert oder substituiert durch Cyan oder $-COOR^{12}$,
$R^{12}$ Wasserstoff, Methyl, Halomethyl, Methoxymethyl, Benzyl oder dasselbe wie $R^9$,
$R^{13}$ und $R^{14}$ unabhängig voneinander je Wasserstoff, $C_1-C_6$-Alkyl oder $C_3-C_6$-Alkenyl,
$R^{15}$ Wasserstoff, $C_1-C_6$-Alkyl oder einen Rest $-COOR^{12}$,
$R^{16}$ und $R^{17}$ unabhängig voneinander je Wasserstoff oder $C_1-C_4$-Alkyl und
Y eine $C_2-C_8$-Alkylengruppe bedeuten,
sowie die Salze dieser Verbindungen mit organischen oder anorganischen Basen.

2. N-(2-Heterocyclylphenylsulfonyl)-N'-pyrimidinyl- und -triazinyl-harnstoffe gemäss Anspruch 1 der Formel Ia

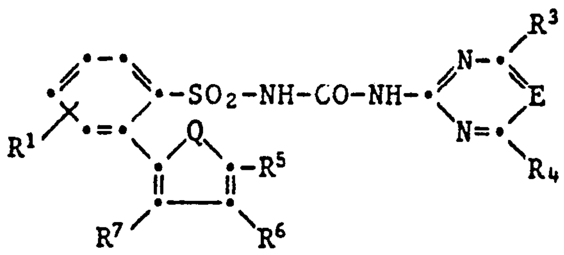

(Ia)

worin E, Q, $R^1$, $R^3$, $R^4$, $R^5$, $R^6$, $R^7$ die im Anspruch 1 gegebene Bedeutung haben.

3. N-(2-Heterocyclylphenylsulfonyl)-N'-pyrimidinyl- und -triazinylharnstoffe gemäss Anspruch 1 der Formel Ia

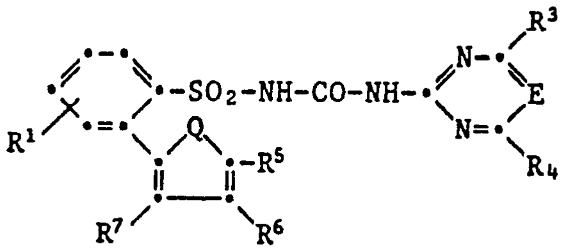

(Ia)

worin
Q Sauerstoff oder Schwefel,
$R^1$ Wasserstoff oder Halogen,
$R^3$ Methyl, Methoxy, Aethoxy, Chlor, Methoxymethyl, Dimethoxymethyl, $C_1$—$C_2$-Halogenalkoxy oder Dimethylamino und
$R^4$ Methyl, Methoxy, Aethoxy, Cyclopropyl oder $OCHF_2$ bedeuten und E und $R^5$, $R^6$ und $R^7$ die im Anspruch 1 angegebenen Bedeutungen haben.

4. N-(2-Heterocyclylphenylsulfonyl)-N'-pyridiminyl- und -triazinyl-harnstoffe gemäss Anspruch 1 der Formel Ib

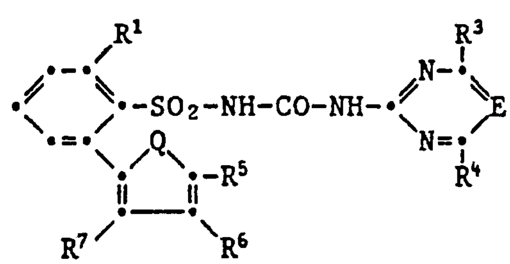

(Ib)

worin
Q Sauerstoff oder Schwefel
$R^1$ Wasserstoff oder Chlor und
$R^3$ und $R^4$ unabhängig voneinander je Methyl oder Methoxy bedeuten und E, $R^5$, $R^6$ und $R^7$ die im Anspruch 1 gegebene Bedeutung haben.

5. N-[2-(5-Acetyl-furan-2-yl)-phenylsulfonyl]-N'-(4,6-dimethoxypyrimidin-2-yl)harnstoff gemäss Anspruch 1.

6. N-[2-(5-Acetyl-furan-2-yl)-phenylsulfonyl]-N'-(4-methoxy-6-methyl-pyrimidin-2-yl)-harnstoff gemäss Anspruch 1.

7. N-(2-Heterocyclylphenylsulfonyl)-N'-pyrimidinyl- und -triazinyl-harnstoffe gemäss Anspruch 1 der Formel Ic

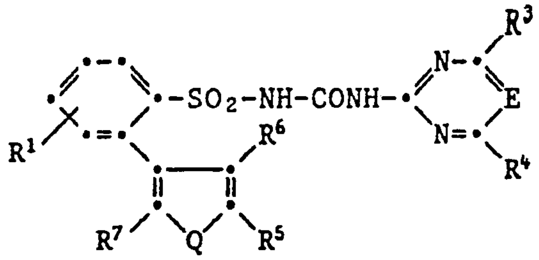

(Ic)

worin E, Q, $R^1$, $R^3$, $R^4$, $R^5$, $R^6$ und $R^7$ die im Anspruch 1 gegebene Bedeutung haben.

8. N-(2-Heterocyclylphenylsulfonyl)-N'-pyrimidinyl- und -triazinyl-harnstoffe gemäss Anspruch 1 der Formel Ic

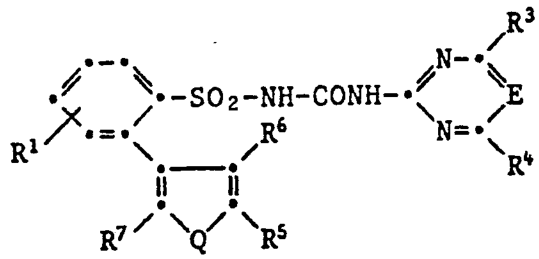

(Ic)

worin

Q Sauerstoff oder Schwefel

$R^1$ Wasserstoff oder Halogen,

$R^3$ Methyl, Methoxy, Aethoxy, Chlor, Methoxymethyl, Dimethoxymethyl, $C_1$—$C_2$-Halogenalkoxy oder Dimethylamino und

$R^4$ Methyl, Methoxy, Aethoxy, Cyclopropyl oder $OCF_2$ bedeuten und E, $R^5$, $R^6$ und $R^7$ die im Anspruch 1 gegebenen Bedeutungen haben.

9. N-(2-Heterocyclylphenylsulfonyl)-N'-pyrimidinyl- und triazinyl-harnstoffe gemäss Anspruch 1 der Formel Id

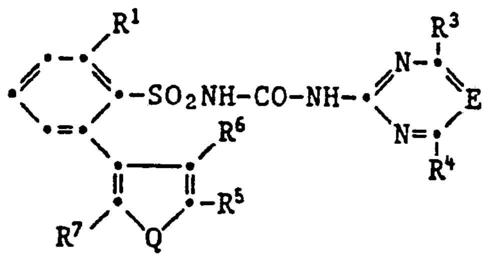

(Id)

worin

Q Sauerstoff oder Schwefel,

$R^1$ Wasserstoff oder Chlor und

$R^3$ und $R^4$ unabhängig voneinander je Methyl oder Methoxy bedeuten und E, $R^5$, $R^6$ und $R^7$ die im Anspruch 1 gegebenen Bedeutung haben.

10. Verfahren zur Herstellung der Verbindungen der Formel I, gemäss Anspruch 1, dadurch gekennzeichnet, dass man ein 2-Heterocyclylphenylsulfonamid der Formel II

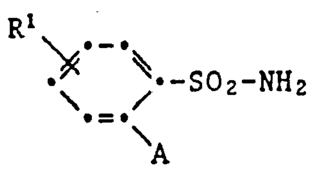

(II),

worin $R^1$ und A die unter Formel I gegebene Bedeutung haben, gegebenenfalls in Gegenwart einer Base mit einem N-Pyrimidinyl- oder -triazinylcarbamat der Formel III

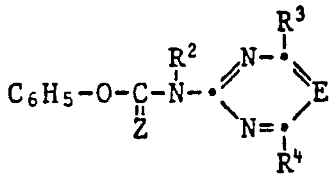

(III),

worin

E, $R^2$, $R^3$, $R^4$ und Z die unter Formel I gegebene Bedeutung haben, umsetzt und gegebenenfalls in die Salze überführt.

11. Verfahren zur Herstellung der Verbindungen der Formel I gemäss Anspruch 1, dadurch gekennzeichnet, dass man ein 2-Heterocyclylphenylsulfonylisocyanat oder -isothiocyanat der Formel IV

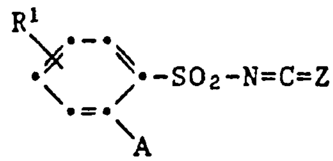

(IV),

worin A, $R^1$ und Z die unter Formel I gegebene Bedeutung haben, gegebenenfalls in Gegenwart einer Base mit einem Aminopyrimidin oder -triazin der Formel V

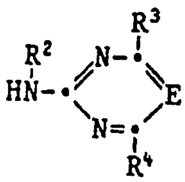

(V)

worin

E, $R^2$, $R^3$ und $R^4$ die unter Formel I gegebene Bedeutung haben, umsetzt und gegebenenfalls in die Salze überführt.

12. Verfahren zur Herstellung der Verbindungen der Formel I gemäss Anspruch 1, dadurch gekennzeichnet, dass man ein 2-Heterocyclylphenylsulfonylcarbamat der Formel VI

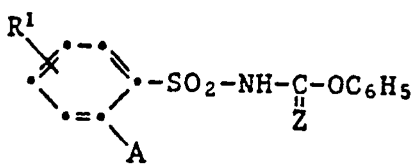

(VI)

worin A, $R^1$ und Z die unter Formel I gegebene Bedeutung haben, mit einem Aminopyrimidin oder -triazin der im Anspruch 11 angegebenen Formel V umsetzt und gegebenenfalls in die Salze überführt.

13. Verfahren zur Herstellung von Additionssalzen der Formel I gemäß Anspruch 1, dadurch gekennzeichnet, dass man einen Sulfonylharnstoff der Formel I mit einem, einem Alkalimetall- oder Erdalkalimetallhydroxid oder einer quaternären Ammoniumbase umsetzt.

14. Ein herbizides und pflanzenwuchshemmendes Mittel, dadurch gekennzeichnet, dass es neben Träger- und/oder anderen Zuschlagstoffen als Wirkstoff mindestens einen substituierten N-Phenylsulfonyl-N'-pyrimidinyl- oder -triazinyl-harnstoff der Formel I, Anspruch 1, enthält.

15. Die Verwendung der Wirkstoffe der Formel I, gemäss Anspruch 1, oder sie enthaltender Mittel zur Bekämpfung unerwünschten Pflanzenwachstums.

16. Die Verwendung der Wirkstoffe der Formel I, gemäss Anspruch 1, oder sie enthaltender Mittel zur Hemmung des Pflanzenwachstums.

17. Die Verwendung der Wirkstoffe der Formel I, gemäss Anspruch 1, oder sie enthaltender Mittel zur Beeinflussung des Pflanzenwachstums zum Zweck der Ertregssteigerung.

18. Die Verwendung der Wirkstoffe der Formel I gemäß Anspruch 1, oder sie enthaltender Mittel zur selektiven pre- oder postemergenten Bekämpfung von Unkräutern in Nutzpflanzenkulturen.

19. Die Verwendung der Wirkstoffe der Formel I gemäß Anspruch 1, oder sie enthaltender Mittel zur Unterdrückung des Pflanzenwachstums über das 2-Blattstadium hinaus, dadurch gekennzeichnet, dass die Wirkstoffe preemergent angewendet werden.

20. 2-Heterocyclylphenylsulfonylderivate der Formel II

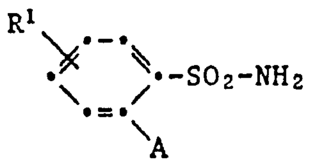

(II),

worin A und $R^1$ die im Anspruch 1 gegebene Bedeutung haben.

21. 2-Heterocyclylphenylsulfonylisocyanate oder -isothiocyanate der Formel IV gemäß Anspruch 11

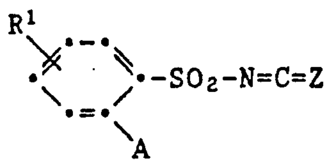

(IV),

22. 2-Heterocyclylphenylsulfonylcarbamate der Formel VI gemäß Anspruch 12

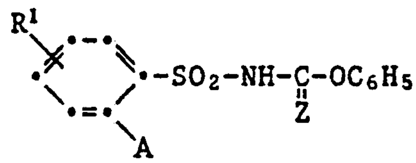

(VI)

worin A, $R^1$ und Z die unter Formel I gegebene Bedeutung haben.

**Revendications**

1-N-(2-hétérocyclyl-phénylsulfonyl)-N'-pyrimidinyl- et -triazinyl-urées de formule I

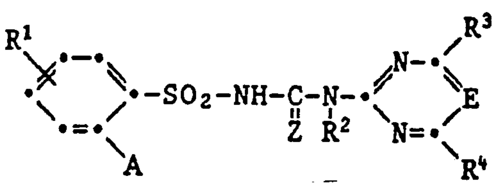

dans laquelle

A représente un groupe

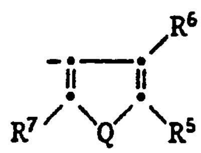

ou

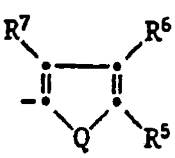

E représente l'azote ou le groupe méthine —CH=,

Q représente l'oxygène, le soufre ou le groupe imino —$NR^8$=,

Z représente l'oxygène ou le soufre,

$R^1$ représente l'hydrogène, un halogène, un groupe nitro, alkyle en $C_1$—$C_4$, halogénoalkyle en $C_1$—$C_4$, alcoxy en $C_1$—$C_4$, halogénoalcoxy en $C_1$—$C_4$, (alcoxy en $C_1$—$C_4$)-carbonyle, alkylthio en $C_1$—$C_4$, alkylsulfinyle en $C_1$—$C_4$, alkylsulfonyle en $C_1$—$C_4$ ou alcoxyalcoxy en $C_2$—$C_5$,

$R^2$ représente l'hydrogène, un groupe alkyle en $C_1$—$C_4$ ou alcoxy en $C_1$—$C_3$,

$R^3$ et $R^4$ représentent chacun, indépendamment l'un de l'autre, l'hydrogène, un halogène, un groupe

alkyle en $C_1$—$C_4$, halogènoalkyle en $C_1$—$C_4$, cycloalkyle en $C_3$—$C_5$, alcoxy en $C_1$—$C_4$, halogénoalcoxy en $C_1$—$C_4$, alkylthio en $C_1$—$C_4$, halogénoalkylthio en $C_1$—$C_4$, alcoxyalkyle en $C_2$—$C_5$, dialcoxyalkyle en $C_3$—$C_6$ ou un groupe amino —$NR^{16}R^{17}$,

$R^5$ représente un groupe alkyle en $C_1$—$C_6$ substitué par des groupes hydroxy, alkylthio en $C_1$—$C_4$, cyano ou un groupe —$COOR^{12}$ ou —$NR^{13}R^{14}$, un groupe alcoxyalcoxy en $C_2$—$C_6$ ou halogénoalcoxy en $C_1$—$C_4$, un groupe halogénoalcényle en $C_2$—$C_6$, un groupe phényle, benzyle, phénoxy ou phénylthio non substitué ou substitué par des groupes alkyle en $C_1$—$C_3$, alcoxy en $C_1$—$C_3$ ou des halogènes, ou bien $R^5$ représente l'un des groupements —$COR^{10}$, —$C(R^{10})=NOR^{11}$ —$CH=CH$—$COOR^{12}$, —$CH=C(COOR^{12})_2$, —$CH=C(CN)$—$COOR^{12}$, —$CR^{10}(OR^{14})_2$,

—$CH_2$—$C(R^{15})$—$COOR^{12}$,
|
$NHCOR^{14}$

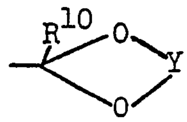

—$NR^{13}$—$COR^{14}$,

$R^6$ et $R^7$ représentent chacun, indépendamment l'un de l'autre, l'hydrogène, un halogène, un groupe alkyle en $C_1$—$C_6$, halogénoalkyle en $C_1$—$C_6$, alcoxyalkyle en $C_2$—$C_6$, alcoxy en $C_1$—$C_6$, alkylthio en $C_1$—$C_4$, di-(alkyle en $C_1$—$C_4$)-amino, cyano, nitro, méthoxycarbonyle ou ont l'une des significations de $R^5$ ou représentent un groupe —$COOR^9$,

$R^8$ représente l'hydrogène, un groupe alkyle en $C_1$—$C_6$, cycloalkyle en $C_3$—$C_6$, benzyle, phényle ou un groupe —$COOR^{12}$,

$R^9$ représente un groupe alkyle en $C_2$—$C_6$ non substitué ou substitué par des halogènes, des groupes alcoxy en $C_1$—$C_4$ ou phényle, un groupe alcényle en $C_3$—$C_6$ ou alcynyle en $C_3$—$C_6$,

$R^{10}$ représente l'hydrogène, un groupe alkyle en $C_1$—$C_6$ non substitué ou substitué par des halogènes, des groupes alcoxy en $C_1$—$C_4$ ou phénoxy lui-même éventuellement substitué par des halogènes, des groupes alkyle en $C_1$—$C_3$ ou alcoxy en $C_1$—$C_3$, ou bien un groupe alcényle en $C_2$—$C_4$,

$R^{11}$ représente l'hydrogène, un groupe alkyle en $C_1$—$C_6$ non substitué ou substitué par des groupes cyano ou —$COOR^{12}$,

$R^{12}$ représente l'hydrogène, un groupe méthyle, halogénométhyle, méthoxyméthyle, benzyle ou a l'une des significations de $R^9$,

$R^{13}$ et $R^{14}$ représentent chacun, indépendamment l'un de l'autre, l'hydrogène, un groupe alkyle en $C_1$—$C_6$ ou alcényle en $C_3$—$C_6$,

$R^{15}$ représente l'hydrogène, un groupe alkyle en $C_1$—$C_6$ ou un groupe —$COOR^{12}$,

$R^{16}$ et $R^{17}$ représentent chacun, indépendamment l'un de l'autre, l'hydrogène ou un groupe alkyle en $C_1$—$C_4$, et

Y représente un groupe alkylène en $C_2$—$C_8$,

ainsi que les sels de ces composés et de bases organiques ou minérales.

2. N-(2-hétérocyclylphénylsulfonyl)-N'-pyrimidinyl- et -triazinyl-urées selon la revendication 1, de formule Ia

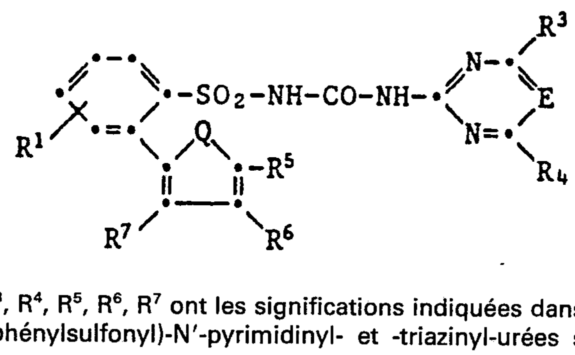
(Ia)

dans laquelle E, Q, $R^1$, $R^3$, $R^4$, $R^5$, $R^6$, $R^7$ ont les significations indiquées dans la revendication 1.

3. N-(2-hétérocyclylphénylsulfonyl)-N'-pyrimidinyl- et -triazinyl-urées selon la revendication 1, de formule Ia

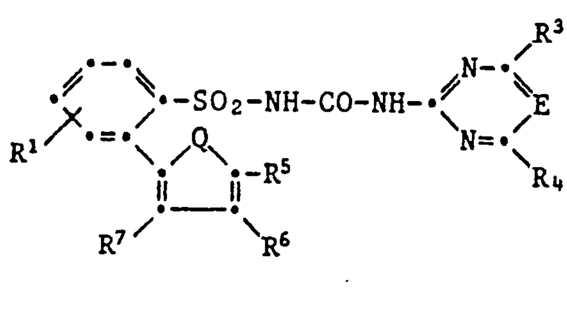
(Ia)

dans laquelle

$Q_1$ représente l'oxygène ou le soufre,

$R^1$ représente l'hydrogène ou un halogène,

$R^3$ représente un groupe méthyle, méthoxy, éthoxy, le chlore, un groupe méthoxyméthyle, diméthyloxyméthyle, halogénoalcoxy en $C_1$—$C_2$ ou diméthylamino, et

$R^4$ représente un groupe méthyle, méthoxy, éthoxy, cyclopropyle ou $OCHF_2$, et

E, $R^5$, $R^6$ et $R^7$ ont les significations indiquées dans la revendication 1.

4. N-(2-hétérocyclylphénylsulfonyl)-N'-pyrimidinyl- et -triazinyl-urées selon la revendication 1, de formule Ib

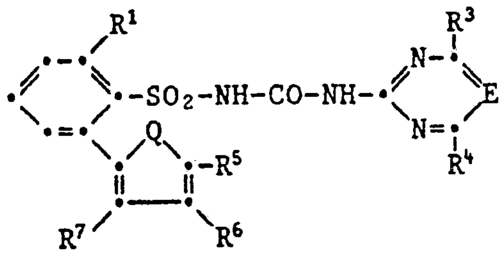

(Ib)

dans laquelle

$Q_1$ représente l'oxygène ou le soufre,

$R^1$ représente l'hydrogène ou le chlore, et

$R^3$ et $R^4$ représentent chacun, indépendamment l'un de l'autre, un groupe méthyle ou méthoxy, et E, $R^5$, $R^6$ et $R^7$ ont les significations indiquées dans la revendication 1.

5. La N-(2-(5-acétyl-furanne-2-yl)phénylsulfonyl]-N'-(4,6-diméthoxy-pyrimidine-2-yl)-urée selon la revendication 1.

6. La N-[2-(5-acétyl-furanne-2-yl)-phénylsulfonyl]-N'-(4-méthoxy-6-méthyl-pyrimidine-2-yl)-urée selon la revendication 1.

7. N-(2-hétérocyclylphénylsulfonyl)-N'-pyrimidinyl- et -triazinyl-urées selon la revendication 1, de formule Ic

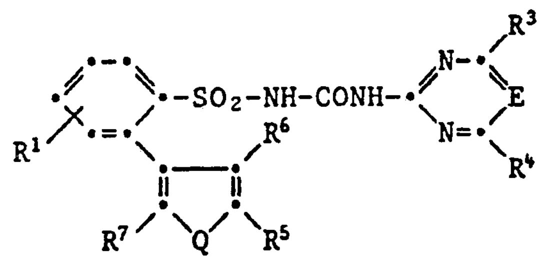

(Ic)

dans laquelle E, Q, $R^1$, $R^3$, $R^4$, $R^5$, $R^6$ et $R^7$ ont les significations indiquées dans la revendication 1.

8. N-(2-hétérocyclylphénylsulfonyl)-N'-pyrimidinyl- et -triazinyl-urées selon la revendication 1, de formule Ic

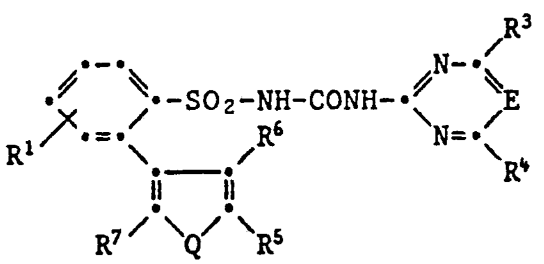

(Ic)

dans laquelle

$Q_1$ représente l'oxygène ou le soufre,

$R^1$ représente l'hydrogène ou un halogène,

$R^3$ représente un groupe méthyle, méthoxy, éthoxy, le chlore, un groupe méthoxyméthyle, diméthoxy-méthyle, halogénoalcoxy en $C_1$—$C_2$ ou diméthylamino, et

$R^4$ représente un groupe méthyle, méthoxy, éthoxy, cyclopropyle ou $OCF_2$, et

E, $R^5$, $R^6$ et $R^7$ ont les significations indiquées dans le revendication 1.

9. N-(2-hétérocyclylphénylsulfonyl)-N'-pyrimidinyl- et -triazinyl-urées selon la revendication 1, de formule Id

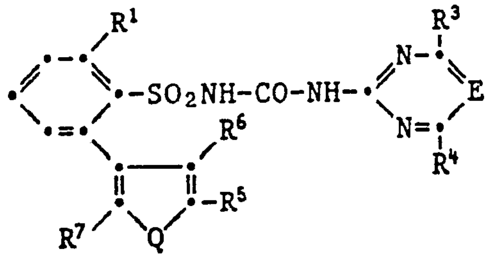

(Id)

dans laquelle
$Q_1$ représente l'oxygène ou le soufre,
$R^1$ représente l'hydrogène ou le chlore, et
$R^3$ et $R^4$ représentent chacun, indépendamment l'un de l'autre, un groupe méthyle ou méthoxy, et
E, $R^5$, $R^6$ et $R^7$ ont les significations indiquées dans la revendication 1.

10. Procédé de préparation des composés de formule I selon la revendication 1, caractérisé en ce que l'on fait réagir un 2-hétérocyclylphénylsulfonamide de formule II

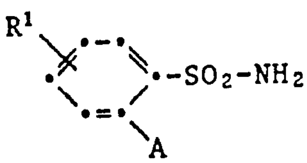

(II),

dans laquelle $R^1$ et A ont les significations indiquées en référece à la formule I, éventuellement en présence d'une base, avec un N-pyrimidinyl- ou -triazinyl-carbamate de formule III

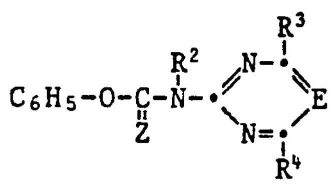

(III),

dans laquelle R, $R^2$, $R^3$, $R^4$ et Z ont les significations indiquées en référence à la formule I, et la cas échéant on convertit en les sels.

11. Procédé de préparation des composés de formule I selon la revendication 1, caractérisé en ce que l'on fait réagir un 2-hétérocyclylphénylsulfonyl-isocyanate ou -isothiocyanate de formule IV

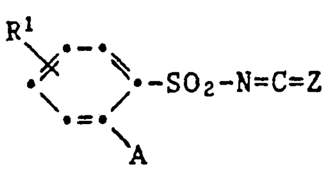

(IV),

dans laquelle A, $R^1$ et Z ont les significations indiquées en référence à la formule I, éventuellement en présence d'une base, avec une amino-pyrimidine ou -triazine de formule V

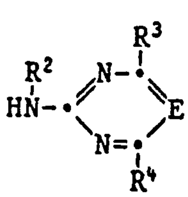

(V)

dans laquelle E, $R^2$, $R^3$ et $R^4$ ont les significations indiquées en référence à la formule I, et le cas échéant, on convertit en les sels.

12. Procédé de préparation des composés de formule I selon la revendication 1, caractérisé en ce que l'on fait réagir un 2-hétérocyclylphénylsulfonylcarbamate de formule VI

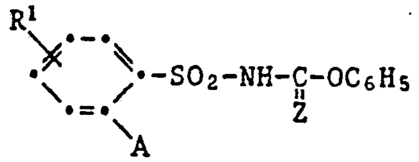

(VI)

dans laquelle A, $R^1$ et Z ont les significations indiquées en référence à la formule I, avec une aminopyrimidine ou -triazine répondant à la formule V de la revendication 11, et le cas échéant on convertit en les sels.

13. Procédé de préparation des sels composés de formule I de la revendication 1 formés par addition, caractérisé en ce que l'on fait réagir une sulfonylurée de formule I avec une amine, un hydroxyde de métal alcalin ou alcalino-terreux ou une base d'ammonium quaternaire.

14. un produit herbicide et régulateur de la croissance des végétaux, caractérisé en ce qu'il contient en tant que substance active, avec des véhicules et/ou d'autres additifs, au moins une N-phénylsulfonyl-N'-pyrimidinyl- ou -triazinyl-urée substituée de formule I, revendication 1.

15. L'utilisation des substances actives de formule I de la revendication 1 ou de produits en contenant pour combattre les croissances de végétaux indésirables.

16. L'utilisation des substances actives de formule I de la revendication 1 ou de produits en contenant pour l'inhibition de la croissance des végétaux.

17. L'utilisation des substance actives de formule I de la revendication 1 ou de produits en contenant pour agir sur la croissance des végétaux en vue d'une augmentation des rendements.

18. L'utilisation des substances actives de formule I de la revendication 1 ou de produits en contenant pour la lutte sélective en pré-levée ou en post-levée contre les végétaux adventices dans les cultures de végétaux utiles.

19. L'utilisation des substances actives de formule I de la revendication 1 ou de produits en contenant pour l'inhibition de la croissance des végétaux au-delà du stade deux feuilles, caractérisée en ce que l'on applique les substances actives en pré-levée.

20. Dérivés 2-hétérocyclylphénylsulfonyliques de formule II

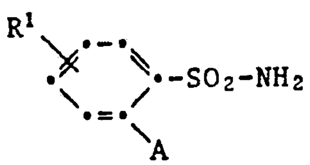

(II),

dans laquelle A et $R^1$ ont les significations indiquées dans la revendication 1.

21. 2-hétérocyclylphénylsulfonyl-isocyanates ou -isothiocyanates de formule IV de la revendication 11

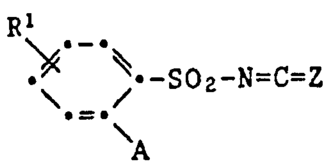

(IV),

dans laquelle A, $R^1$ et Z ont les significations indiquées en référence à la formule I.

22. 2-hétérocyclylphénylsulfonylcarbamates de formule VI de la revendication 12.

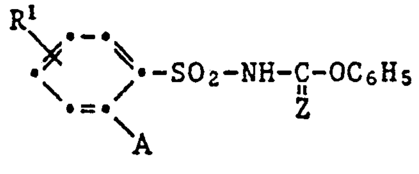

(VI)

dans laquelle A, $R^1$ et Z ont les significations indiquées en référence à la formule I.

**Claims**

1. An N-(2-heterocyclylphenylsulfonyl)-N'-pyrimidinylurea or N-(2-heterocyclylphenylsulfonyl)-N'-triazinylurea of formula I

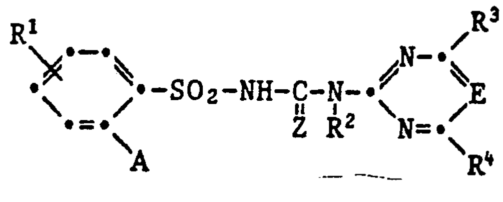

in which

A is a

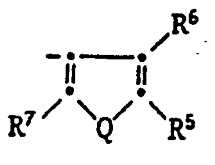

or

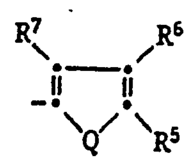

radical,

E is nitrogen or the methane group $-CH=$,

Q is oxygen, sulfur or the imino group $-NR^8=$,

Z is oxygen or sulfur,

$R^1$ is hydrogen, halogen, nitro, $C_1-C_4$alkyl, $C_1-C_4$haloalkyl, $C_1-C_4$alkoxy, $C_1-C_4$haloalkoxy, $C_1-C_4$-alkoxycarbonyl, $C_1-C_4$alkylthio, $C_1-C_4$alkylsulfinyl, $C_1-C_4$alkylsulfonyl or $C_2-C_5$alkoxyalkoxy,

$R^2$ is hydrogen, $C_1-C_4$alkyl or $C_1-C_3$alkoxy,

$R^3$ and $R^4$ are each independently of the other hydrogen, halogen, $C_1-C_4$alkyl, $C_1-C_4$haloalkyl, $C_3-C_5$cycloalkyl, $C_1-C_4$alkoxy, $C_1-C_4$haloalkoxy, $C_1-C_4$alkylthio, $C_1-C_4$haloalkylthio, $C_2-C_5$alkoxyalkyl, $C_3-C_6$dialkoxyalkyl or an amino radical $-NR^{16}R^{17}$,

$R^5$ is a $C_1-C_6$alkyl radical which is substituted by hydroxyl, $C_1-C_4$alkylthio, cyano or a $-COOR^{12}$ group or $-NR^{13}R^{14}$, or is $C_2-C_6$alkoxyalkoxy or $C_1-C_4$haloalkoxy, $C_2-C_6$haloalkenyl, or a phenyl, benzyl, phenoxy or phenylthio radical which is unsubstituted or substituted by $C_1-C_3$alkyl, $C_1-C_3$alkoxy or halogen, or $R^5$ is one of the groupings $-COR^{10}$, $-C(R^{10})=NOR^{11}$, $-CH=CH-COOR^{12}$, $-CH=C(COOR^{12})_2$, $-CH=C(CN)COOR^{12}$, $-CR^{10}(OR^{14})_2$,

$-CH_2-C(R^{15})COOR^{12}$,
|
$NHCOR^{14}$

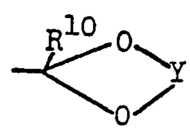

or $-NR^{13}-COR^{14}$,

$R^6$ and $R^7$ are each independently of the other hydrogen, halogen, $C_1-C_6$alkyl, $C_1-C_6$haloalkyl, $C_2-C_6$-alkoxyalkyl, $C_1-C_6$alkoxy, $C_1-C_4$alkylthio, di($C_1-C_4$alkyl)amino, cyano, nitro, methoxycarbonyl or the same as $R^5$ or $-COOR^9$,

$R^8$ is hydrogen, $C_1-C_6$alkyl, $C_3-C_6$cycloalkyl, benzyl, phenyl or a $-COOR^{12}$ radical,

$R^9$ is a $C_2-C_6$alkyl radical which is unsubstituted or substituted by halogen, $C_1-C_4$alkoxy or phenyl, or is $C_3-C_6$alkenyl or $C_3-C_6$alkynyl,

$R^{10}$ is hydrogen, $C_1-C_6$alkyl which is unsubstituted or substituted by halogen, $C_1-C_4$alkoxy or phenoxy which may in turn be substituted by halogen, $C_1-C_3$alkyl or $C_1-C_3$alkoxy, or $R^{10}$ is $C_2-C_4$alkenyl,

$R^{11}$ is hydrogen or $C_1-C_6$alkyl which is unsubstituted or substituted by cyano or $-COOR^{12}$,

$R^{12}$ is hydrogen, methyl, halomethyl, methoxymethyl, benzyl or the same as $R^9$,

$R^{13}$ and $R^{14}$ are each independently of the other hydrogen, $C_1-C_6$alkyl or $C_3-C_6$alkenyl,

$R^{15}$ is hydrogen, $C_1-C_6$alkyl or a $-COOR^{12}$ radical,

$R^{16}$ and $R^{17}$ are each independently of the other hydrogen or a $C-C_4$alkyl, and

Y is a $C_2-C_8$alkylene group,

or a salt of such a compound with an organic or inorganic base.

2. An N-(2-heterocyclylphenylsulfonyl)-N'-pyrimidinylurea or N-(2-heterocyclylphenylsulfonyl)-N'-triazinylurea according to claim 1 of formula Ia

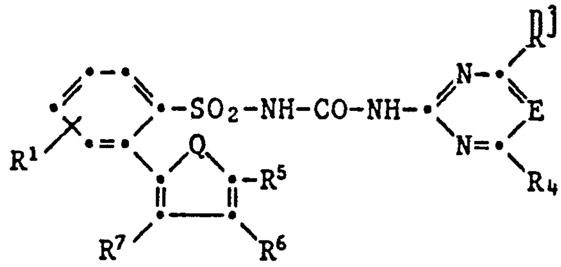

(Ia)

in which E, Q, $R^1$, $R^3$, $R^4$, $R^5$, $R^6$ and $R^7$ are as defined in claim 1.

3. An N-(2-heterocyclylphenylsulfonyl)-N'-pyrimidinylurea or N-(2-heterocyclylphenylsulfonyl)-N'-triazinylurea according to claim 1 of formula Ia

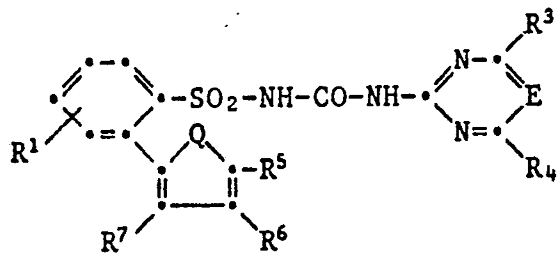

(Ia)

in which

Q is oxygen or sulfur,

$R^1$ is hydrogen or halogen,

$R^3$ is methyl, methoxy, ethoxy, chlorine, methoxymethyl, dimethoxymethyl, $C_1$—$C_2$haloalkoxy or dimethylamino, and

$R^4$ is methyl, methoxy, ethoxy, cyclopropyl or $OCHF_2$, and

E and $R^5$, $R^6$ and $R^7$ are as defined in claim 1.

4. An N-(2-heterocyclylphenylsulfonyl)-N'-pyrimidinylurea or N-(2-heterocyclylphenylsulfonyl)-N'-triazinylurea according to claim 1 of formula Ib

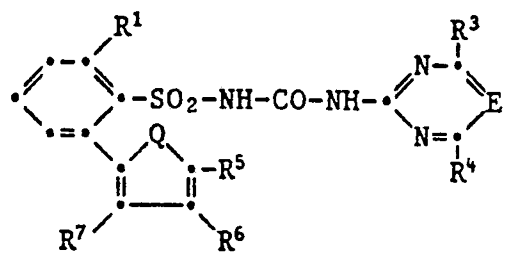

(Ib)

in which

Q is oxygen or sulfur,

$R^1$ is hydrogen or chlorine, and

$R^3$ and $R^4$ are each independently of the other methyl or methoxy, and E, $R^5$, $R^6$ and $R^7$ are as defined in claim 1.

5. N-[2-(5-Acetylfuran-2-yl)phenylsulfonyl]-N'-(4,6-dimethoxypyrimidin-2-yl)urea according to claim 1.

6. N-[2-(5-Acetylfuran-2-yl)phenylsulfonyl]-N'-4-methoxy-6-methylpyrimidin-2-yl)urea according to claim 1.

7. An N-(2-heterocyclylphenylsulfonyl)-N'-pyrimidinylurea or N-(2-heterocyclylphenylsulfonyl)-N'-triazinylurea according to claim 1 of formula Ic

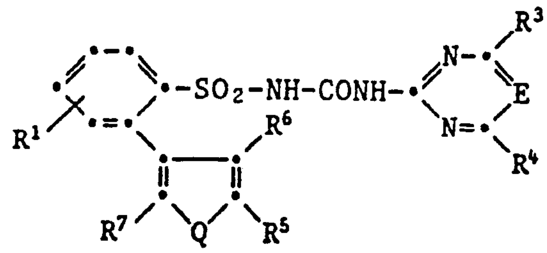

(Ic)

in which E, Q, $R^1$, $R^3$, $R^4$, $R^5$, $R^6$ and $R^7$ are as defined in claim 1.

8. An N-(2-heterocyclylphenylsulfonyl)-N'-pyrimidinylurea or N-(2-heterocyclylphenylsulfonyl)-N'-triazinylurea according to claim 1 of formula Ic

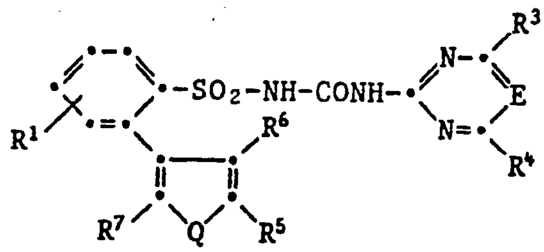

(Ic)

in which

Q is oxygen or sulfur,

$R^1$ is hydrogen or halogen,

$R^3$ is methyl, methoxy, ethoxy, chlorine, methoxymethyl, dimethoxymethyl, $C_1$—$C_2$haloalkoxy or dimethylamino, and

$R^4$ is methyl, methoxy, ethoxy, cyclopropyl or $OCF_2$, and

E, $R^5$, $R^6$ and $R^7$ are as defined in claim 1.

9. An N-(2-heterocyclylphenylsulfonyl)-N'-pyrimidinylurea or N-(2-heterocyclylphenylsulfonyl)-N'-triazinylurea according to claim 1 of formula Id

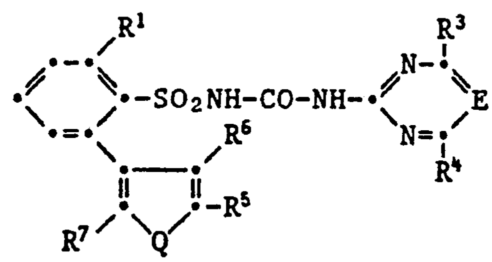

(Id)

in which

Q is oxygen or sulfur,

$R^1$ is hydrogen or chlorine, and

$R^3$ and $R^4$ are each independently of the other methyl or methoxy, and E, $R^5$, $R^6$ and $R^7$ are as defined in claim 1.

10. A process for the preparation of a compound of formula I according to claim 1, which process comprises reacting a 2-heterocyclylphenylsulfonamide of formula II

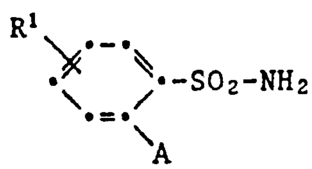

(II),

in which $R^1$ and A are as defined for formula I, with an N-pyrimidinyl carbamate or N-triazinyl carbamate of formula III

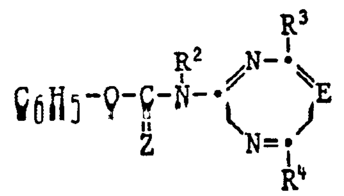

(III),

in which E, $R^2$, $R^3$, $R^4$ and Z are as defined for formula I, optionally in the presence of a base, and optionally converting said compound of formula I into a salt thereof.

11. A process for the preparation of a compound of formula I according to claim 1, which process comprises reacting a 2-heterocyclylphenylsulfonyl isocyanate or 2-heterocyclylphenylsulfonyl isothiocyanate of formula IV

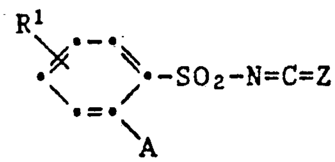

(IV),

in which A, $R^1$ and Z are as defined for formula I, with an aminopyrimidine or aminotriazine of formula V

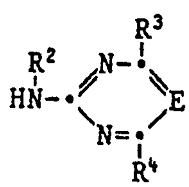

(V)

in which E, $R^2$, $R^3$ and $R^4$ are as defined for formula I, optionally in the presence of a base, and optionally converting said compound of formula I into a salt thereof.

12. A process for the preparation of a compound of formula I according to claim 1, which process comprises reacting a 2-heterocyclylphenylsulfonyl carbamate of formula VI

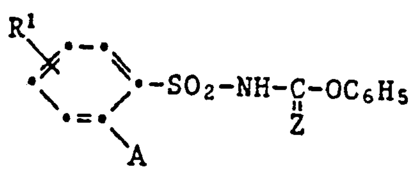

(VI)

in which A, $R^1$ and Z are as defined for formula I, with an aminopyrimidine or aminotriazine of formula V as indicated in claim 11, and optionally converting said compound of formula I into a salt thereof.

13. A process for the preparation of an addition salt of formula I according to claim 1, which process comprises reacting a sulfonylurea of formula I with an amine, an alkali metal hydroxide or an alkaline earth metal hydroxide or with a quaternary ammonium base.

14. A herbicidal and plant growth regulating composition which contains, as active compound, at least one substituted N-phenylsulfonyl-N'-pyrimidinylurea or N-phenylsulfonyl-N'-triazinylurea of formula I according to claim 1, together with carriers and/or other adjuvants.

15. The use of an active compound of formula I according to claim 1, or of a composition containing such a compound, for controlling undesired plant growth.

16. The use of an active compound of formula I according to claim 1 or of a composition containing such a compound, for inhibiting plant growth.

17. The use of an active compound of formula I according to claim 1, or of a composition containing such a compound, for influencing plant growth for increasing yield.

18. The use of an active compound of formula I according to claim 1, or of an agent containing such a compound, for selectively controlling weeds, pre- or post-emergence in groups of useful plants.

19. The use of an active compound of formula I according to claim 1, or of a composition containing such a compound, for suppressing plant growth beyond the two-leaf stage, wherein the active compound is applied preemergence.

20. A 2-heterocyclylphenylsulfonyl derivative of formula II

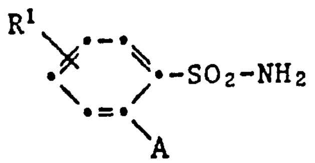

(II),

in which A and $R^1$ are as defined in claim 1.

21. A 2-heterocyclylphenylsulfonyl isocyanate or 2-heterocyclylphenylsulfonyl isothiocyanate of formula IV according to claim 11

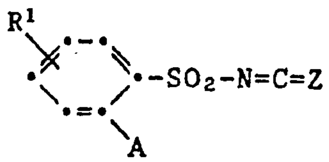

(IV),

in which A, $R^1$ and Z are as defined for formula I.

22. A 2-heterocyclylphenylsulfonyl carbamate of formula VI according to claim 12

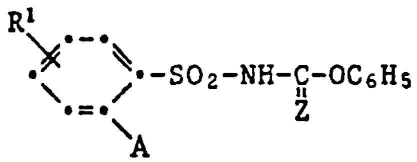

(VI)

in which A, $R^1$ and Z are as defined for formula I.